# EUROPEAN PATENT SPECIFICATION

(11) **EP 2 329 819 B1**
(45) Date of publication and mention of the grant of the patent: **01.05.2019**
(21) Application number: 09811170.1
(22) Date of filing: 04.09.2009
(51) Int. Cl.: A61K 31/4196, A61K 31/4168, A61P 31/10, A61P 33/04

(54) **PHARMACEUTICAL COMPOSITION FOR USE IN TREATING SEXUALLY TRANSMITTED INFECTIONS**
PHARMAZEUTISCHE ZUSAMMENSETZUNG ZUR BEHANDLUNG VON SEXUELL ÜBERTRAGBAREN INFEKTIONEN
COMPOSITION PHARMACEUTIQUE DESTINÉE À ÊTRE UTILISÉE DANS LE TRAITEMENT DES INFECTIONS SEXUELLEMENT TRANSMISSIBLES

(30) Priority: 04.09.2008 MX 2008011321
(43) Date of publication of application: 08.06.2011
(73) Proprietor: Laboratorios Senosiain, S.A. De C.V., Distrito Federal 11560 (MX)
(72) Inventor: GARCÍA SALGADO LÓPEZ, Raùl, 11320 Mexico D.F. (MX); ARZOLA PANIAGUA, Angélica, 11320 Mexico D.F. (MX); SENOSIAIN PELÁEZ, Juan Pablo, 11320 Mexico D.F. (MX); BARRANCO HERNÁNDEZ, Gustavo, 11320 Mexico D.F. (MX)
(74) Representative: Carpintero Lopez, Francisco
(86) International application number: PCT/IB2009/006738
(87) International publication number: WO 2010/026469

(56) References cited:
- EP-A1- 1 712 229
- WO-A2-2007/079391
- MX-A- PA02 007 641
- PEPIN JACQUES ET AL: "The syndromic management of vaginal discharge using single-dose treatments: a randomized controlled trial in West Africa", BULLETIN OF THE WORLD HEALTH ORGANIZATION, vol. 84, no. 9, September 2006 (2006-09), pages 729-738, XP009150897, ISSN: 0042-9686
- MASHBURN ET AL: "Etiology, Diagnosis, and Management of Vaginitis", JOURNAL OF MIDWIFERY & WOMEN'S HEALTH, ELSEVIER, vol. 51, no. 6, 1 November 2006 (2006-11-01), pages 423-430, XP025149537, ISSN: 1526-9523, DOI: DOI:10.1016/J.JMWH.2006.07.005 [retrieved on 2006-11-01]
- DATABASE BIOSIS [Online] BIOSCIENCES INFORMATION SERVICE, PHILADELPHIA, PA, US; 1998, ANKIRSKAYA A S ET AL: "Diflucan-150: Experience with the treatment and prevention of vaginal candidiasis", XP002655070, Database accession no. PREV199800343591
- R.VILLAGRANA-ZESATI ET AL.: 'Short-term therapy for mixed vaginal infections' INTERNATIONAL JOURNAL OF GYNECOLOGY AND OBSTETRICS vol. 92, 2006, pages 149 - 150, XP025240683
- M. MALHOTRA ET AL.: 'Ciprofloxacin-tinidazol combination, fluconazole-azithromicin- secnidazole-kit and doxycycline-metronidazole combination therapy in syndromic management of pelvic inflammatory disease: A prospective randomized controlled trial' INDIAN JOURNAL OF MEDICAL SCIENCES vol. 57, no. 12, 2003, pages 549 - 555, XP003001449
- VILLAGRANA-ZESATI JESÚS ROBERTO ET AL: "[Clinical efficacy of fluconazole, tinidazole and clindamycin vs fluconazole, tinidazole and azithromycin in the treatment of mixed cervical-vaginal infections, included those caused by Mycoplasma and Chlamydia trachomatis].", GINECOLOGIA Y OBSTETRICIA DE MEXICO MAY 2013, vol. 81, no. 5, May 2013 (2013-05), pages 231-238, ISSN: 0300-9041

## Description

### FIELD OF THE INVENTION.

The present invention refers to a solid oral pharmaceutical composition comprising the combination of fluconazole, tinidazole and clindamycin and/or pharmaceutically acceptable salts thereof, wherein the content of the pharmaceutically acceptable excipients is significantly lower than the content of active agents; and to said composition for oral administration with therapeutic activity for use in the treatment of sexually transmitted infections.

### BACKGROUND OF THE INVENTION

Genital infections are one of the most common problems in gynecology, they are a group of infectious diseases caused by different types of microorganisms, whose common denominator is that they are mainly acquired through sexual intercourse and are called sexually transmitted infections (STIs).

These diseases do not have uniform effects, some can be serious, causing chronic pain, sterility, infertility, inflammatory pelvic disease; if they occur during pregnancy they may cause abortion, premature birth, ectopic pregnancy, among others. Therefore, if not prevented, diagnosed or treated early, they contribute to increased morbidity and mortality of women and men.

Prevalence studies about sexually transmitted infections (STIs) submitted by Villagrana-Zesati in "Sexually Transmitted Infectious Diseases, 2008" indicate that the two main infections are candidiasis and bacterial vaginosis, with incidences of 39% and 30% respectively, followed by infections whose etiologic agents are *Ureaplasma urealyticum, Chlamydia trachomatis* and *Trichomonas vaginalis.* It is worth to mention that mixed infection ranks third in frequency of infections, associating Candidiasis with Bacterial vaginosis or Candidiasis with Trichomoniasis.

Other reports from several national and foreign authors, indicate that within the fungal infections, *Candida albicans* is the most frequent agent, and within bacterial infections the most common is bacterial vaginosis followed by other organisms such as mycoplasma, *Chlamydia* and *Trichomonas.*

**Table 1. Major diseases caused by sexually transmitted infections.**

| Infection | Causative Agent and Clinical Manifestation | Treatment (Regular Recommended Dose) |
|---|---|---|
| | | - Butaconazol 2%; vaginal cream for 3 days. |
| Vulvovaginal Candidiasis | The main causative agent is *Candida* albicans; Others that may occur: *C. glabrata,* *C*. *topicalis, C. parapsilosis.* | |
| | | - Clotrimazole 1%; vaginal cream or tablet 7 to 14 days. |
| | | - Clotrimazole vaginal tablet; two tablets/day for 3 days. |
| | Clinical manifestations include: itching, burning, vulvar edema, fissures and abrasions. | - Isoconazole 1%; vaginal cream for 7 days |
| | | - Itraconazole 200mg for 3 days. |
| | | - Miconazole 2%; vaginal cream for 7 days. |
| | The vaginal discharge is white, thick, lumpy, it is observed as attached to the cervix, to the vaginal walls and to the vulva. | |
| | | - Nystatin 100000 I.U.; vaginal tablet. One tablet for 14 days. |
| | | - Tioconazole 6.5%; vaginal cream, one dose. |
| | | - Terconazole 0.4; vaginal cream 0.4% for 7 days; 0.8% for 3 days. |
| | | - Fluconazole 150mg, tablet; a single dose. |
| Bacterial vaginosis | The main causative agent is *Gardnerella vaginalis* and additionally anaerobic Gram-negative bacilli. | Antibiotics: |
| | | - Clindamycin cream 2%, a daily dose for 5 days; |
| | | - Clindamycin 300mg capsule; twice a day for 7 days; |
| | Clinical manifestations: leukorrhea (whitish-gray, homogeneous fluid, in moderate or heavy amount). | |
| | | - Clindamycin ovules 100mg; a daily dose for 3 days. |
| | | - Metronidazole; 2 capsules/day for 7 days; gel application for 7 days. |
| Chlamydiasis | The main causative agent is Gram positive bacteria *Chlamydia* in three species: *C*. *Trachomatis, C. muridarum and C.suis* | - Azithromycin 1g, single oral dose |
| | | - Clindamycin HCl, 450mg, 4 doses/day for 10 or 14 days |
| | | - Erythromycin 500mg, capsule, 4 doses daily for 7 days. |
| | It is a strict intracellular bacteria. | - Ofloxacin, 300mg capsule, twice a day for 7 days. |
| | Clinical manifestations: May be asymptomatic. Sequels may result in serious infections, such as pelvic inflammatory disease, ectopic pregnancy and infertility. | - Levofloxacin 500mg, capsule, a daily dose for 7 days. |
| | | - Doxycycline 100 mg, capsule, twice a day for 7 days. |
| Trichomoniasis | The main causative agent is the protozoan *Trichomona vaginalis* | -Metronidazole capsule, 2g, single dose; 500mg twice a day for 7 days. |
| | | - Tinidazole, 2g capsule, a single dose |

*Chlamydia trachomatis* is a strict intracellular bacteria, thus it was considered as a virus at the beginning of its identification. This condition obstructs its study because it cannot be cultivated on artificial media, therefore the use of cell cultures is the most suitable way for studying it, though it is not the most accessible one. Another technique consists in direct immunofluorescence. Currently, the Food and Drug Administration (FDA) approved the use of nucleic acid hybridization, because it proved to be the most sensitive.

The disease caused by *Chlamydia* is often asymptomatic, or the symptoms are so mild that many patients that have the disease do not realize it until they develop a more severe complication. It is estimated that 75% of women and 50% of men who are infected with *Chlamydia* do not notice any symptoms.

The symptoms in women, when present, are the following: abnormal leukorrhea, abnormal vaginal bleeding, pain or burning when urinating or pain in the lower abdomen, especially during sexual intercourse. Infection by *Chlamydia* in men can cause: testicular swelling or hypersensitivity, possibly symptoms of epididymitis which can cause infertility, urethritis, internal infection of the penis that can cause pain and urinating difficulty.

Mixed vulvovaginitis infection is the result of the association of two or more microorganisms, for example, the presence of candidiasis and bacterial vaginosis or candidiasis and trichomoniasis. In the case of mixed infection treatments, formulations have been developed, combining two active agents in short treatment schemes of 3 days up to the commonly used 7 days, as illustrated in Table 2.

**Table 2. Combined Scheme for Treating Mixed Vulvovaginitis Infection**

| Active Agent | Dosage Form | Dose |
|---|---|---|
| Fluconazole-Tinidazole | Oral Tablet 150 mg /2,000 mg | Single dose |
| Itraconazole-Secnidazole | Oral Capsules 33.3 mg /166.6 mg | Every 12 hours for 3 days |
| Tioconazole-Tinidazole | Vaginal tablet 100 mg /150 mg | Every 12 hours for 3 days |
| Clindamycin-Ketoconazole | Ovules 100 mg /400 mg | 1 daily dose for 7 days |

PEPIN et al. ("The syndromic management of vaginal discharge using single-dose treatments: a randomized controlled trial in West Africa", BULLETIN OF THE WORLD HEALTH ORGANIZATION, 2006, vol. 84, no. 9, pages 729-738) disclose that once daily oral combination of fluconazole and tinidazole is effective in the treatment of sexually transmitted vulvovaginal infections.

ANKIRSKAYA et al. ("Diflucan-150: Experience with the treatment and prevention of vaginal candidiasis", DATABASE BIOSIS; BIOSCIENCES INFORMATION SERVICE, PHILADELPHIA, PA, US; 1998, Database accession no. PREV199800343591; XP002655070) disclose fluconazole for use in the treatment and prevention of vaginal candidiasis.

One of the agents used for treating vaginal infections is clindamycin, which is an antibiotic of the lincosamide family. It is a semisynthetic antibiotic that has a bacteriostatic effect and interferes with protein synthesis. Clindamycin has a half-life of 21 hours. Active clindamycin and its metabolites are excreted mainly in urine and some in bile.

Clindamycin is more effective against infections involving the following types of organisms:
- Aerobic gram-positive cocci, including some staphylococci and streptococci (e.g. pneumococci).
- Anaerobic gram-negative bacilli, including some members of the Bacteroides genera and Fusobacterium genera.
- It attacks infections caused by *Chlamydia.*

MASHBURN et al. ("Etiology, Diagnosis, and Management of Vaginitis", JOURNAL OF MIDWIFERY & WOMEN'S HEALTH, ELSEVIER, 2006, vol. 51, no. 6, pages 423-430) disclose a 7 days oral treatment of sexually transmitted diseases with clindamycin. EP 1712229 A1 discloses a combination of tinidazole and fluconazole for use in a one-day treatment of mixed infectious diseases in the human reproductive system. It also discloses clindamycin for use in the treatment of sexually transmitted diseases, such as *Gardnerella,* wherein such treatment requires the oral administration of 300 mg clindamycin, twice a day, for a week.

Clindamycin may cause transitory adverse effects, although they can be moderately important. The most common effects are: nausea, vomiting, diarrhea, abdominal pain, flatulence, skin rash, itching.

Azithromycin is another antibiotic used against uncomplicated sexually transmitted infections due to *Chlamydia Trachomatis.*

In the state of the art, U.S. patent 7,094,431 refers to a pharmaceutical composition for treating skin repair, preferably by topical application, although oral administration is also mentioned. This formulation can be administered orally or not orally, and may contain: zinc oxide, fat soluble vitamins (A, D, E and K), an antibacterial agent, an antifungal agent and an effective amount of a calcium channel blocker such as nifedipine.

Unlike this document, the present invention is characterized by being an oral pharmaceutical composition comprising the combination of the active ingredients fluconazole, tinidazole and clindamycin, for use in the treatment of mixed vulvovaginal infection and sexually transmitted diseases in a daily dose.

US20050165077 patent document and its equivalent PA/a/0608279, refer to a composition containing fluconazole, tinidazole or secnidazole for treating vaginal infections of the type *Gardnerella vaginalis, Actinomyces, Candida, Micrococcus,* yeasts, *Proteus, E. Coli, Trichomonas vaginalis,* microorganisms present in vaginitis and bacterial vaginosis.

Unlike this document, the present invention addresses a broad spectrum of fungal and bacterial infections, and those caused by aerobic or anaerobic microorganisms such as *Chlamydia, Gardnerella vaginalis,* gram-negative and gram-positive bacilli and cocci, *Peptostreptococcus,* genital mycoplasmas *and mobiluncus,* with a high percentage of eradication and effectiveness in a very short scheme of treatment.

The novel combination of fluconazole, tinidazole and clindamycin has not been reported to date. In the state of the art there are several studies that include combinations of drugs that do not fully address, and without a high degree of effectiveness, the conditions of sexually transmitted infections or mixed vulvovaginal infections.
- Malhotra (2003) worked on combinations of ciprofloxacin, and tinidazole applied for seven days and fluconazole-azithromycin-secnidazole in the form of a kit, and the combination of doxycycline and metronidazole for several days.
- Ariella Baylson (2004) conducted a study about the treatment with tinidazole for recurrent infections caused by bacterial vaginosis.
- Livengood (2007) studied two tinidazole treatment schemes for bacterial vaginosis.
- Say, P (2005) conducted studies about the difficulty of treating vaginitis.

Unlike these studies for the treatment of vaginal infections, the present invention refers to an oral pharmaceutical composition of fluconazole, tinidazole and clindamycin, for use in the treatment of sexually transmitted infections. This composition is applied in a single day, has a wide spectrum and results in a decrease of infectious relapse. The sexually transmitted infection is selected from the group consisting of candidiasis, trichomoniasis, mycoplasma, chlamydiasis, bacterial vaginosis, diseases caused by *Gardnerella vaginalis* and mixed vulvovaginal infections. Clindamycin or its pharmaceutically acceptable salts are in a total dose of 300 to 2400 mg.
- Workowski, K.A. et. *al* (2006, 55 RR11) in their document "Sexually Transmitted Disease Treatment Guidelines. Morbidity and Mortality Weekly Report 2006; 55, RR11", presents a guide for the treatment of sexually transmitted infections (STIs). In this guide there are individual and combined treatments that eradicate several mixed vulvovaginal-cervical conditions. However, the time period for the combined treatment is more than one day and additionally the guide does not mention the use of the combination of fluconazole, tinidazole and clindamycin.

The novel combination of fluconazole, tinidazole and clindamycin has not been reported to date. The present pharmaceutical invention exhibits significant advantages over existing formulations for STIs, such as:
▪ A broad-spectrum effect, because the combination allows the physician to safely and reliably attack (in pharmaceutical terms) the mixed vulvovaginosis infections without having to wait for the laboratory test results.
▪ Decreasing the possibility of failure due to discontinuation of treatment, because it involves a simplified scheme of a single day treatment.
▪ The one day treatment is more easily accepted by the couple as compared with other treatments that last more than one day, such as the treatment with clindamycin, which according to the prior art, is administered at least twice a day for 7 days.
▪ The combination is completely absorbed in the gastrodigestive tract, which allows achieving appropriate plasma concentrations without any competition between the active ingredients. This effect occurs with or without food ingestion, this results in no limitations at the moment of administering the product.
▪ The fluconazole-tinidazole-clindamycin combination does not interact in the process of elimination, or in the average life of each one of the active ingredients. It was found that this allows for an adequate contribution of therapeutic effects in the combination, without compromising patient health.
▪ The pharmaceutical composition has at least one active agent in an amount less than the regular total dose, as is the case of using a lower dose of clindamycin in a single day treatment, which leads to fewer adverse effects.
▪ The combination is stable and meets pharmaceutical specifications such as: appearance, content uniformity, dissolution, valuation; this is achieved despite the fact that the composition contains a higher content of active agents than the content of excipients.
▪ In the present invention, the pharmaceutical composition is physicochemically stable and meets pharmaceutical specifications. In a preferred embodiment, the content of excipients is not greater than 40% by weight of the dosage unit.

### JUSTIFICATION OF THE INVENTION

The need to attack a broad spectrum of microorganisms by combining fluconazole, tinidazole and clindamycin is socially useful because it can be used in villages that lack of the technical or economic conditions for performing clinical tests, and that only count on medical diagnosis for treatment.

Surprisingly, in the present invention, the composition with the association of fluconazole, tinidazole and clindamycin is therapeutically effective, although clindamycin is at a significantly lower total dose compared with the regular doses, and although it is administered in a one-day treatment. This fact improves the scheme of attention of these diseases with respect to the known treatments. The therapeutic scope for the spectrum of microorganisms includes *Gardnerella vaginalis* and *Chlamydia,* which results in a significant advantage over longer-term treatments because it ensures treatment compliance by the patient.

The regular oral dose of clindamycin for the treatment of vaginal infections is 300mg to 450mg per day for 7 days or more. However, in the present invention, clindamycin is administered in a one-day treatment, at total doses of 300 mg to 2400 mg, a preferred total dose might be 1200 mg to 1800 mg.

A technical challenge overcome by this invention is the association of fluconazole, tinidazole and clindamycin or suitable pharmaceutically salts thereof in a single unit dose and a lower content of excipients.

Additionally, this composition offers a highly therapeutic effectiveness that allows a rapid reduction of the symptoms, it is well tolerated and more acceptable by the patient.

Another challenge overcome by the present invention is to offer the combination of fluconazole, tinidazole and clindamycin in a dosage unit of an acceptable size so that it can be swallowed by the patient.

For the aforementioned reasons, the present invention provides a pharmaceutical composition for preparing a drug for use in the treatment of sexually transmitted infections using the combined effect of the active agents of the composition.

The therapeutic effectiveness provided by the combination of fluconazole, tinidazole and clindamycin against infections such as bacterial vaginosis, candidiasis, chlamydiasis, tricomoniaisis, mycoplasma, and/or mixed vulvovaginitis, makes this invention an effective alternative for use in the treatment of these conditions with a therapeutic scheme of a single day treatment.

The present invention offers an oral formulation, preferably tablets, where is important to highlight that the active agents fluconazole, tinidazole and clindamycin do not have good compression properties nor fluidity. One would expect that during the development of the formulation, it may be necessary to add excipients in order to compensate the lack of compression and fluidity.

Evidently, if less excipients are used, it is more difficult to achieve fluidity and compressibility. Additionally, a lower percentage of excipients in the formulation reduces the possibility of modifying physical properties such as appearance, consistency, dissolution rate, content uniformity.

In the present invention it is possible to obtain a physicochemically stable formulation that meets fluid compressibility specifications, appearance, content uniformity, dissolution rate, and which contains a smaller amount of excipients, for example, below 40%.

### SUMMARY OF THE INVENTION

The present invention is defined by the appended claims. Thus, the invention refers to:
1.- An oral pharmaceutical composition characterized by comprising tinidazole, fluconazole and clindamycin or pharmaceutically acceptable salts thereof and pharmaceutically acceptable excipients, for use in the treatment of sexually transmitted infections selected from the group consisting of candidiasis, trichomoniasis, mycoplasma, chlamydiasis, bacterial vaginosis, diseases caused by *Gardnerella vaginalis* and mixed vulvovaginal infections, wherein clindamycin or its pharmaceutically acceptable salts are in a total dose of 300 to 2400 mg, and wherein the treatment is a one-day treatment.
2.- The pharmaceutical composition according to claim 1 for use according to claim 1, wherein clindamycin or its pharmaceutically acceptable salts are in a total dose of 1200-1800 mg.
3.- The pharmaceutical composition according to claim 1 for use according to claim 1, wherein clindamycin or its pharmaceutically acceptable salts are in a total dose of 1250 mg.
4.- The pharmaceutical composition according to any one of the preceding claims for use according to claim 1, wherein the composition is in solid form.
5.- The pharmaceutical composition according to any one of the preceding claims for use according to claim 1, wherein the composition is in tablet form.
6.- The pharmaceutical composition according to claim any one of the preceding claims for use according to claim 1, comprising the following pharmaceutically acceptable excipients or at least one of the following excipients: microcrystalline cellulose, sodium starch glycolate, crospovidone, sodium lauryl sulfate, polyvinylpyrrolidone, magnesium stearate, methacrylate derivatives (Opadry White) and isopropyl alcohol.

### DETAILED DESCRIPTION OF THE INVENTION

The formulation and administration of drug combinations is not easy, because when administering two or more active ingredients in a single dosage form, interactions between the active agents, adverse reactions, side effects, incompatibility (physic, chemical and physicochemical), as well as technological problems resulting from the physicochemical interaction of the active agents themselves and with the excipients, may occur.

In accordance with the above description, it can be seen that the broad spectrum, the treatment shortness and the effectiveness of the active ingredients combination, as well as the security of having a pharmaceutical form with all the criteria of quality, are factors of great importance for giving benefits to the patients with an appropriate treatment for these diseases.

### FORMULATIONS

The formulation and manufacturing process of the pharmaceutical composition of fluconazole, tinidazole and clindamycin or pharmaceutically acceptable salts thereof, additionally, pharmaceutically acceptable carriers or excipients, are described below:
Generally, for the tablet formulation, the active ingredients fluconazole, tinidazole and clindamycin do not exhibit suitable fluidity and compressibility properties.

In the present invention is possible to obtain a physicochemically stable formulation that meets the specifications of formulation stability.

To manufacture the present invention, different excipients were tested, such as: compressibility carriers, microcrystalline cellulose, lactose, starch; diluent binders, lactose, dextrose, sucrose, mannitol, polividone; antistatics, sodium lauryl sulfate, silicon dioxide, talc; disintegrants, croscarmellose sodium, crospovidone, sodium starch glycolate; lubricants, magnesium stearate, magnesium phosphate, stearic acid, glyceryl stearate, polyethylene glycol, sodium stearyl fumarate, talc; plasticizers, propylene glycol, polyethylene glycol, among other glycol derivatives; ligand polymer binders, hypromellose, polyvinyl pirrolidone, hydroxypropyl cellulose; coating polymers, methacrylate copolymer derivatives (Opadry), polyvynilpirrolidone, hydroxypropylmethylcellulose; coating and finishing polymer, cellulose and methacrylate derivatives (Opaglos), polyvinylpirrolidone, polyvinyl alcohol, polyethylene glycol, hydroxypropylmethylcellulose. Within all the excipients, equivalent excipients and/or mixtures thereof were tested.

### FORMULATION EXAMPLE

Table 3 presents a general formulation of the triple combination. Tables 4 and 5 illustrate examples of formulations comprising fluconazole, tinidazole and clindamycin, where the amounts by weight of active ingredients, carriers and/or excipients may be used within the mentioned ranges.

**Table 3. General oral tablet formulation**

| COMPONENTS | RANGE OF USE WEIGHT PERCENT PER UNIT DOSE |
|---|---|
| Tinidazole | 40 to 55% |
| Fluconazole | 1 to 4% |
| Clindamycin | 30 to 65% |
| Compressibility carriers | 2 to 12% |
| Diluent binder | 0.1 to 2.6% |
| Disintegrant | 0.05 to 4% |
| Antistatic | 0.1 to 1% |
| Binding ligand polymer | 1.5 to 4% |
| Lubricant | 0.2 to 0.4% |
| Coating and finishing polymer | 0.0 to 2% |
| Isopropyl alcohol | --- |
| Purified water | qs |

**Table 4. Formulation 1, oral tablets with clindamycin.**

| Components | Weight Percent per Unit Dose | Content (mg) per Unit Dose |
|---|---|---|
| Tinidazole | 50 | 500 mg |
| Fluconazole | 3.7 | 37.5 mg |
| Clindamycin (as clindamycin hydrochloride) | 32.5 | 312.5 mg |
| Microcrystalline cellulose PH 102 | 7.5 | 85 mg |
| Sodium starch glycolate | 0.6 | 6.5 mg |
| Crospovidone | 1.6 | 17 mg |
| Sodium lauryl sulfate | 0.6 | 7 mg |
| Polyvinylpyrrolidone | 1.9 | 20 mg |
| Magnesium stearate | 0.3 | 3.2 mg |
| Methacrylate derivatives (Opadry White) | 1 | 10.5 mg |
| Isopropyl alcohol | --- | --- |
| Purified water | qs | qs |

**Table 5. Formulation 2, oral tablets with clindamycin.**

| Components | Weight Percentage/Unit Dose | Content Mg/Unit Dose |
|---|---|---|
| Tinidazole | 40 | 500 mg |
| fluconazole | 3 | 37.5 mg |
| Clindamycin (as clindamycin hydrochloride) | 36 | 450 mg |
| Microcrystalline cellulose pH 102 | 10.4 | 130 mg |
| Sodium starch glycolate | 2 | 25 mg |
| Crospovidone | 3.8 | 48 mg |
| Sodium lauryl sulfate | 0.8 | 10 mg |
| Polyvinylpyrrolidone | 3.6 | 45 mg |
| Magnesium stearate | 0.3 | 3.7 mg |
| Isopropyl Alcohol | --- | --- |
| Purified water | qs | qs |

### MANUFACTURING PROCESS

The manufacturing process for the combination of active agents in a single solid phase is a novel method for the incorporation of fluconazole, a wet granulation in which tinidazole is incorporated and clindamycin for further compression.
1) Formulation components are weighed.
2) Binder solution is prepared by dissolving in water: isopropyl alcohol, fluconazole, polyvinyl pyrrolidone, sodium lauryl sulfate.
3) Sieved apart: Tinidazole, third active principle, microcrystalline cellulose, sodium starch glycolate and crospovidone.
4) The materials from step 3 are mixed in a "V" mixer.
5) The mixture obtained in step 4 is granulated in a fluidized-bed equipment, with the binder solution of step 2.
6) The obtained granulate is sieved.
7) Crospovidone and sodium lauryl sulfate are added to the granulate.
8) The mixture from step 7 is granulated.
9) Magnesium stearate is added to the mixture from step 8 and then mixed.
10) The mixture from step 9 is compressed to obtain a tablet.
11) The coating is prepared separately, by adding water to the methacrylate derivative.
12) The coating is applied on the tablets obtained in step 11.
13) The obtained product is conditioned.

The process can be used with other solid oral compositions which can be, without limitation, granules and capsules. In fact, the maximum values of the use ranges shown in Table 3 are considered suitable for granulates. The process for such granulation is the same as the one previously mentioned for preparing tablets, but without the tabletting steps.

The tablets were submitted to a stability evaluation, the results are shown in Table 6.

**Table 6. Physicochemical Evaluation of the Formulations**

| **Formulation** | **Evaluation Time** | **Appearance** | **Dose Uniformity** | **Weight Percent per Unit Dose** |
|---|---|---|---|---|
| 1 | Starting point | Oblong tablet with no stains or spots | Complies | 99% |
| 2 | Three months | Oblong tablet with no stains or spots | Complies | 98% |

As previously described, it is observed that the tablets comply with stability requirements.

The combination of fluconazole - tinidazole - clindamycin is effective for use in the treatment of the most common germs found in the clinical practice of the reproductive tract, such as *Candida albicans, Gardnerella vaginalis, Chlamydia, Trichomonas vaginalis,* among others.

The composition with fluconazole-tinidazole-clindamycin combination, has fewer side effects due to the lower amount of clindamycin used (1250 mg), compared to the regular recommended dose for treatment.

The invention has been sufficiently described so that a person of ordinary skill in the subject matter can reproduce and obtain the results mentioned in this description.

## Claims

1. An oral pharmaceutical composition **characterized by** comprising tinidazole, fluconazole and clindamycin or pharmaceutically acceptable salts thereof and pharmaceutically acceptable excipients, for use in the treatment of sexually transmitted infections selected from the group consisting of candidiasis, trichomoniasis, mycoplasma, chlamydiasis, bacterial vaginosis, diseases caused by *Gardnerella vaginalis* and mixed vulvovaginal infections, wherein clindamycin or its pharmaceutically acceptable salts are in a total dose of 300 to 2400 mg, and wherein the treatment is a one-day treatment.

2. The pharmaceutical composition according to claim 1 for use according to claim 1, wherein clindamycin or its pharmaceutically acceptable salts are in a total dose of 1200-1800 mg.

3. The pharmaceutical composition according to claim 1 for use according to claim 1, wherein clindamycin or its pharmaceutically acceptable salts are in a total dose of 1250 mg.

4. The pharmaceutical composition according to any one of the preceding claims for use according to claim 1, wherein the composition is in solid form.

5. The pharmaceutical composition according to any one of the preceding claims for use according to claim 1, wherein the composition is in tablet form.

6. The pharmaceutical composition according to claim any one of the preceding claims for use according to claim 1, comprising the following pharmaceutically acceptable excipients or at least one of the following excipients: microcrystalline cellulose, sodium starch glycolate, crospovidone, sodium lauryl sulfate, polyvinylpyrrolidone, magnesium stearate, methacrylate derivatives (Opadry White) and isopropyl alcohol.

## Patentansprüche

1. Orale pharmazeutische Zusammensetzung, **dadurch gekennzeichnet, dass** sie Tinidazol, Fluconazol und Clindamycin oder pharmazeutisch verträgliche Salze davon und pharmazeutisch verträgliche Hilfsstoffe umfasst, zur Verwendung bei der Behandlung von sexuell übertragbaren Infektionen, ausgewählt aus der Gruppe bestehend aus Candidiasis, Trichomoniasis, Mycoplasma, Chlamydiasis, bakterieller Vaginose, durch *Gardnerella vaginalis* verursachten Krankheiten und verschiedenen vulvovaginalen Infektionen, wobei Clindamycin oder die pharmazeutisch verträglichen Salze davon in einer Gesamtdosis von 300 bis 2400 mg sind, und wobei die Behandlung eine Eintagsbehandlung ist.

2. Pharmazeutische Zusammensetzung nach Anspruch 1 zur Verwendung nach Anspruch 1, wobei Clindamycin oder die pharmazeutisch verträglichen Salze davon in einer Gesamtdosis von 1200-1800 mg sind.

3. Pharmazeutische Zusammensetzung nach Anspruch 1 zur Verwendung nach Anspruch 1, wobei Clindamycin oder die pharmazeutisch verträglichen Salze davon in einer Gesamtdosis von 1250 mg sind.

4. Pharmazeutische Zusammensetzung nach einem der vorgehenden Ansprüche zur Verwendung nach Anspruch 1, wobei die Zusammensetzung in fester Form ist.

5. Pharmazeutische Zusammensetzung nach einem der vorgehenden Ansprüche zur Verwendung nach Anspruch 1, wobei die Zusammensetzung in Tablettenform ist.

6. Pharmazeutische Zusammensetzung nach einem der vorhergehenden Ansprüche zur Verwendung nach Anspruch 1, umfassend die folgenden pharmazeutisch verträglichen Hilfsstoffe oder zumindest einen von den folgenden Hilfsstoffen: mikrokristalline Cellulose, Natriumstärkeglycolat, Crospovidon, Natriumlaurylsulfat, Polyvinylpyrrolidon, Magnesiumstearat, Methacrylatderivaten (Opadry White) und Isopropylalkohol.

## Revendications

1. Composition pharmaceutique pour une administration orale **caractérisée en ce qu'**elle comprend du tinidazole, du fluconazole et de la clindamycine ou leurs sels pharmaceutiquement acceptables et des excipients pharmaceutiquement acceptables, pour une utilisation dans le traitement d'infections sexuellement transmissibles choisies dans le groupe constitué par la candidose, la tricomonase, l'infection mycoplasmique, la chlamydiose, la vaginose bactérienne, les maladies causées par *Gardnerella vaginalis* et les vulvo-vaginites mixtes, dans laquelle la clindamycine ou ses sels pharmaceutiquement acceptables sont à une dose totale de 300 à 2400 mg, et dans laquelle le traitement est un traitement d'un jour.

2. Composition pharmaceutique selon la revendication 1 pour une utilisation selon la revendication 1, dans laquelle la clindamycine ou ses sels pharmaceutiquement acceptables sont à une dose totale de 1200-1800 mg.

3. Composition pharmaceutique selon la revendication 1 pour une utilisation selon la revendication 1, dans laquelle la clindamycine ou ses sels pharmaceutiquement acceptables sont à une dose totale de 1250 mg.

4. Composition pharmaceutique selon l'une quelconque des revendications précédentes pour une utilisation selon la revendication 1, dans laquelle la composition est sous forme solide.

5. Composition pharmaceutique selon l'une quelconque des revendications précédentes pour une utilisation selon la revendication 1, dans laquelle la composition est sous forme de comprimé.

6. Composition pharmaceutique selon l'une quelconque des revendications précédentes pour une utilisation selon la revendication 1, comprenant les excipients pharmaceutiquement acceptables suivants ou au moins un des excipients suivants: la cellulose microcristalline, le glycolate d'amidon sodique, la crospovidone, le laurylsulfate de sodium, la polyvinylpyrrolidone, le stéarate de magnésium, les dérivés de méthacrylate (Opadry White) et l'alcool isopropylique.
